# EUROPEAN PATENT SPECIFICATION

(11) **EP 3 796 846 B1**
(45) Date of publication and mention of the grant of the patent: **18.03.2026**
(21) Application number: 19806743.1
(22) Date of filing: 21.05.2019
(51) Int. Cl.: A61B 10/00, A61B 42/10, A61B 46/23, A61M 25/00, A61M 25/18, A61M 27/00

(54) **URINE-SAMPLING KIT**
URINPROBENAHMEKIT
KIT D'ÉCHANTILLONNAGE D'URINE

(30) Priority: 22.05.2018 US 201862675112 P
(43) Date of publication of application: 31.03.2021
(73) Proprietor: C. R. Bard, Inc., Franklin Lakes, NJ 07417 (US)
(72) Inventor: WAITKUS, Tim, Sparta, NJ 07871 (US); FODOUOP, Chris, Atlanta, GA 30312 (US); LEEKE, Kelsey, Atlanta, GA 30306 (US); GOHDE, John, Decatur, GA 30030 (US)
(74) Representative: Hoffmann Eitle
(86) International application number: PCT/US2019/033382
(87) International publication number: WO 2019/226693

(56) References cited:
- US-A1- 2003 195 478
- US-A1- 2007 025 886
- US-A1- 2007 161 971
- US-A1- 2012 172 822
- US-A1- 2014 188 002
- US-A1- 2015 290 425
- US-A1- 2016 054 202
- US-A1- 2017 216 558
- US-A1- 2017 216 558
- US-A1- 2018 057 196
- US-B1- 6 520 948

## Description

### BACKGROUND

Hospitals around the country are understandably focusing their efforts on catheter-associated urinary tract infection ("CAUTI") prevention bundles to achieve better outcomes, limit the risk of Centers for Medicare & Medicaid Services ("CMS") reimbursement penalties, and reduce the occurrence of antimicrobial resistance. Historical prevention efforts have focused on closed systems, aseptic insertion technique, and maintenance of Foley- catheterized patients. However, despite 96% of nursing decision makers believing there is variation in how nurses within their facilities take urine samples from Foley catheters, there has been little to no broad-based focus to date on the significant variation associated with urine sampling practice.

Research suggests there is variation in all aspects of urine sampling including where the urine sample is taken from the collection system, how the urine-sampling area is cleaned, what device is used to take the urine sample, and how the urine sample is transferred to the lab. This variation is a fundamental issue affecting documentation of CAUTI outcomes. In fact, 100% of nursing decision makers believe variation and improper urine-sampling technique can lead to an increased risk of contamination and therefore false-positive CAUTIs. Moreover, up to 70% of urine cultures reflect false-positive results leading to inaccurate CAUTI diagnoses and inappropriate antibiotic treatments, as well as artificially undermining the time and resources hospitals have dedicated toward reducing the risk of CAUTI by other means. This problem presents an ongoing challenge to those seeking to reduce CAUTI rates to avoid CMS reimbursement penalties.

Disclosed herein are urine-sampling kits and methods thereof that address at least the foregoing.

US 2016/0054202 A1 discloses a diurnal urine collection system. In one embodiment, a collection system includes a first collection container, a second collection container, a funnel, and a diverter valve. The diverter valve is coupled to the first collection container, the second collection container, and the funnel. The diverter valve may have a first position configured to direct the liquid sample from the funnel into the first collection container, and a second position configured to direct the liquid sample from the funnel into the second collection container.

US 6,520,948 B1 discloses a sampling tube holder for blood sampling system. The holder has an interior pocket which can be conformed from a closed position to an open position for receiving the sampling tube.

US 2014/0188002 A1 discloses an infusion and blood collection device and method.

US 2017/0216558 A1 discloses a catheter insertion tray with integrated instructions. An improved medical procedure or catheterization tray is included in an improved medical procedure or catheterization package. The improved medical procedure or catheterization tray is intuitively arranged and includes instructions printed thereon to improve the medical procedure or catheterization implementation and results. In one example, a catheterization package and catheterization tray has a layout and/or arrangement of components that may help reduce CAUTI rates by facilitating ease of use and aiding in proper aseptic technique during insertion. The medical procedure or catheterization package and/or medical procedure or catheterization tray may include various implements, compartments, and components necessary and/or helpful to the medical procedure or catheterization, including, for example, improved swabs and an improved compartment for holding the swabs.

US 2004/0195478 A1 discloses a closed system irrigation connector for urinary catheters.

US 2007/0025886 A1 discloses a body fluid collecting, transporting, and dispensing system which includes a body fluid collecting receptacle and a handle for supporting the receptacle in a fluid collection position. The handle includes a fluid sample chamber in fluid communication with the receptacle to receive a sample of the body fluid and the handle is further removable from the receptacle for transport and dispensing of the body fluid sample through a tip. A flexible hollow streaker, attachable to the tip, enables streaking of fluid sample from the chamber onto an agar plate or the like.

### SUMMARY OF THE INVENTION

In a first aspect of the invention, there is provided a catheterization-and-urine-sampling kit according to claim 1.

The dependent claims define preferred embodiments.

### SUMMARY OF THE DISCLOSURE

Disclosed herein is a urine-sampling kit including a sampling-port access device, a package of hand sanitizer, a pair of sterile gloves, and a packaged antiseptic-saturated material having a cleansing form configured for cleansing. The sampling-port access device includes a barrel, a tip at an end of the barrel configured to fluidly connect the sampling-port access device to a urine-sampling port of a catheter assembly, and a hollow needle coaxial with the barrel. The needle is fluidly connected to but directed away from the tip of the barrel. Contents of the urine-sampling kit including the sampling-port access device, the package of hand sanitizer, the pair of sterile gloves, and the packaged antiseptic-saturated material having the cleansing form are packaged in accordance with step-by-step instructions for aseptic urine sampling.

In some embodiments, the urine-sampling kit further includes a fenestrated drape for placement over a patient. A fenestration of the fenestrated drape is configured to allow access to a urine-sampling port of a catheter assembly when the fenestrated drape is draped over the patient.

In some embodiments, the urine-sampling kit further includes a tubing clamp configured to clamp drainage tubing between a urine-sampling port and a drainage bag of a catheter assembly. Clamping the drainage tubing with the tubing clamp allows urine to back up into the urine-sampling port for urine sampling.

The urine-sampling kit further includes one or more septum-stoppered test tubes. Each test tube of the one or more test tubes has an internal pressure less than atmospheric pressure.

Each test tube of the one or more test tubes independently includes therein a formulation for urinalysis, or a formulation for microbiological analysis, wherein the formulation for urinalysis includes chlorhexidine, ethylparaben, or sodium propionate and wherein the formulation for microbiological analysis includes boric acid, sodium formate, or sodium borate.

In some embodiments, the urine-sampling kit further includes central supply room ("CSR") wrap wrapped around contents of the urine-sampling kit. The CSR wrap is configured to preserve a sterile state of the contents of the urine-sampling kit while wrapped around the contents of the urine-sampling kit.

In some embodiments, the urine-sampling kit further includes a molded tray including one or more compartments configured to hold the contents of the urine-sampling kit.

In some embodiments, the urine-sampling kit further includes an outer packaging of the urine-sampling kit. The outer packaging is configured to protect the urine sampling kit from damage and loss of the components of the urine-sampling kit from a point of manufacturing the urine-sampling kit to a point of using the urine-sampling kit.

In some embodiments, the urine-sampling kit further includes a molded tray, CSR wrap, and an outer packaging. The molded tray includes one or more compartments configured to hold the contents of the urine-sampling kit. The CSR wrap is configured to preserve a sterile state of the contents of the urine-sampling kit while the CSR wrap is wrapped around the molded tray and the contents of the urine-sampling kit. The outer packaging of the urine-sampling kit is configured to protect the urine-sampling kit from damage and loss of the components of the urine-sampling kit from a point of manufacturing the urine-sampling kit to a point of using the urine-sampling kit.

Also disclosed herein is a catheterization-and-urine-sampling kit including a set of catheterization components and a set of urine-sampling components. The set of catheterization components includes a pre-connected catheter assembly including a urinary catheter, drainage tubing, and a connector fluidly connecting the urinary catheter to the drainage tubing. The connector includes a urine-sampling port. The set of urine-sampling components includes a sampling-port access device, a package of hand sanitizer, a pair of sterile gloves, and a packaged antiseptic-saturated material having a cleansing form configured for cleansing. The sampling-port access device includes a tip at an end of a barrel configured to fluidly connect the sampling-port access device to the urine-sampling port of the catheter assembly. Contents of the urine-sampling kit including the sampling-port access device, the package of hand sanitizer, the pair of sterile gloves, and the packaged antiseptic-saturated material having the cleansing form are packaged in accordance with step-by-step instructions for aseptic urine sampling.

In some embodiments, each of the urine-sampling port of the connector and the tip of the sampling-port access device has a complementary thread pattern to the other for locking the sampling-port access device onto the urine-sampling port.

In some embodiments, the catheterization-and-urine-sampling kit further includes a first fenestrated drape and a second fenestrated drape. The first fenestrated drape is provided for placement over a patient. A fenestration of the first fenestrated drape is shaped and sized to allow access to the patient for insertion of the urinary catheter when the first fenestrated drape is draped over the patient. The second fenestrated drape is also provided for placement over the patient. A fenestration of the second fenestrated drape is shaped and sized to allow access to the urine-sampling port when the second fenestrated drape is draped over the patient.

In some embodiments, the catheterization-and-urine-sampling kit further includes a bedsheet clip for securing the drainage tubing to one or more bedsheets. The bedsheet clip is also configured to clamp the drainage tubing between the urine-sampling port and a drainage bag of the catheter assembly. Clamping the drainage tubing with the tubing clamp allows urine to back up into the urine-sampling port for urine sampling.

The catheterization-and-urine-sampling kit further includes one or more septum-stoppered test tubes. Each test tube of the one or more test tubes has an internal pressure less than atmospheric pressure. Each test tube of the one or more test tubes is also independently configured to include therein a formulation for urinalysis, or a formulation for microbiological analysis. The formulation for urinalysis includes chlorhexidine, ethylparaben, or sodium propionate and wherein the formulation for microbiological analysis includes boric acid, sodium formate, or sodium borate.

The sampling-port access device includes a hollow needle coaxial with the barrel of the sampling-port access device, wherein the needle is fluidly connected to but directed away from the tip of the barrel. In some embodiments, each test tube of the one or more test tubes has an outer diameter commensurate with or smaller than an inner diameter of the barrel of the sampling-port access device. Sized as such, each test tube is configured to slide into the barrel of the sampling-port access device to pierce the septum stopper of the test tube with the needle of the sampling-port access device for urine sampling.

In some embodiments, the catheterization-and-urine-sampling kit further includes CSR wrap wrapped around the contents of the catheterization-and-urine-sampling kit. The CSR wrap is configured to preserve a sterile state of the contents of the catheterization- and-urine-sampling kit while wrapped around the contents of the catheterization-and-urine- sampling kit.

In some embodiments, the catheterization-and-urine-sampling kit further includes a molded tray including one or more compartments configured to hold the contents of the catheterization-and-urine-sampling kit.

In some embodiments, the catheterization-and-urine-sampling kit further includes an outer packaging of the catheterization-and-urine-sampling kit. The outer packaging is configured to protect the catheterization-and-urine-sampling kit from damage and loss of the components of the catheterization-and-urine-sampling kit from a point of manufacturing the catheterization-and-urine-sampling kit to a point of using the catheterization-and-urine- sampling kit.

In some embodiments, the catheterization-and-urine-sampling kit further includes a molded tray, CSR wrap, and an outer packaging. The molded tray includes one or more compartments configured to hold the contents of the catheterization-and-urine-sampling kit. The CSR wrap is configured to preserve a sterile state of the contents of the catheterization- and-urine-sampling kit while the CSR wrap is wrapped around the molded tray and the contents of the catheterization-and-urine-sampling kit. The outer packaging of the catheterization-and- urine-sampling kit is configured to protect the catheterization-and-urine-sampling kit from damage and loss of the components of the catheterization-and-urine-sampling kit from a point of manufacturing the catheterization-and-urine-sampling kit to a point of using the catheterization-and-urine-sampling kit.

Also disclosed herein, but not encompassed by the scope of the claims, is a method for aseptic urine sampling including removing an outer packaging from a urine-sampling kit, aseptically unwrapping the urine-sampling kit, cleaning a urine-sampling port of a catheter assembly, fluidly connecting a sampling-port access device to the urine-sampling port, and aspirating a urine sample from the urine-sampling port. Removing the outer packaging from the urine-sampling kit includes exposing one or more steps of a set of step-by-step instructions incorporated into the urine-sampling kit for the urine sampling. Unwrapping the urine-sampling kit includes forming a sterile working surface of an inner surface of CSR wrap for the urine sampling. Unwrapping the urine-sampling kit also includes exposing additional steps of the step-by-step instructions as well as sterile contents of the urine-sampling kit disposed over the sterile working surface. The sterile contents of the urine-sampling kit include at least the sampling-port access device and a packaged antiseptic- saturated material having a cleansing form configured for cleansing. The sampling-port access device includes a tip at an end of a barrel configured to fluidly connect the sampling-port access device to the urine-sampling port of the catheter assembly. Cleaning the urine-sampling port or the catheter assembly includes cleaning the urine-sampling port with the antiseptic-saturated material having the cleansing form.

The method for aseptic urine sampling further includes obtaining one or more septum-stoppered test tubes and sliding at least one test tube of the one or more test tubes into the barrel of the sampling-port access device. The one or more septum stoppered test tubes are optionally obtained from the urine-sampling kit if provided therein. Each test tube of the one or more test tubes has an internal pressure less than atmospheric pressure. Each test tube of the one or more test tubes independently includes therein a formulation for urinalysis, or a formulation for microbiological analysis. Sliding the at least one test tube of the one or more test tubes into the barrel of the sampling-port access device pierces the septum stopper of the test tube with a hollow needle disposed in the barrel of the sampling-port access device. Once the needle pierces the septum stopper of the test tube, urine is automatically aspirated into the test tube from the urine sampling port due to the less-than-atmospheric internal pressure of the test tube to provide a urine sample. The formulation for urinalysis includes chlorhexidine, ethylparaben, or sodium propionate and wherein the formulation for microbiological analysis includes boric acid, sodium formate or sodium borate.

The method for aseptic urine sampling further includes clamping drainage tubing of the catheter assembly with a tubing clamp or a bedsheet clip between the urine-sampling port and a drainage bag of a catheter assembly. Clamping the drainage tube with the tubing clamp or bedsheet clip allows urine to back up into the urine sampling port for urine sampling.

The method for aseptic urine sampling further includes aseptically removing a package of hand sanitizer provided in the urine-sampling kit from the sterile working surface and subsequently using the hand sanitizer for hand antisepsis. In addition, the method for aseptic urine sampling further includes removing a pair of sterile gloves provided in the urine-sampling kit from the sterile working surface and subsequently donning the pair of sterile gloves.

The method for aseptic urine sampling further includes donning a pair of exam gloves before removing the outer packaging from the urine-sampling kit. Because the pair of exam gloves has a potential to get contaminated by the outer packaging of the urine-sampling kit, the method for aseptic urine sampling further includes removing the pair of exam gloves after removing the outer packaging from the urine-sampling kit. Optionally following additional hand washing or hand antisepsis, the method for aseptic urine sampling
further includes aseptically unwrapping the urine-sampling kit to expose the sterile contents of the urine-sampling kit.

### DRAWINGS

FIG. 1 provides a schematic illustrating an unopened urine-sampling kit in accordance with some embodiments.
FIG. 2A provides a schematic illustrating a first unwrapped urine-sampling kit in accordance with some embodiments.
FIG. 2B provides a schematic illustrating a second unwrapped urine-sampling kit in accordance with some embodiments.
FIG. 3 provides a schematic illustrating an exclusive connection between a sampling-port access device and a urine-sampling port of a connector in accordance with some embodiments.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein. The scope of the invention is defined by the appended claims solely.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "front," "back," "top," "bottom," "forward," "reverse," "clockwise," "counter clockwise," "up," "down," or other similar terms such as "upper," "lower," "aft," "fore," "vertical," "horizontal," "proximal," "distal," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal end portion" of, for example, a catheter disclosed herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal end portion, or the proximal length of the catheter can include the proximal end of the catheter; however, the proximal portion, the proximal end portion, or the proximal length of the catheter need not include the proximal end of the catheter. That is, unless context suggests otherwise, the proximal portion, the proximal end portion, or the proximal length of the catheter is not a terminal portion or terminal length of the catheter.

With respect to "distal," a "distal portion" or a "distal end portion" of, for example, a catheter disclosed herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal end portion, or the distal length of the catheter can include the distal end of the catheter; however, the distal portion, the distal end portion, or the distal length of the catheter need not include the distal end of the catheter. That is, unless context suggests otherwise, the distal portion, the distal end portion, or the distal length of the catheter is not a terminal portion or terminal length of the catheter.

Unless defined otherwise, all technical and scientific terms used herein have the same meaning as commonly understood by those of ordinary skill in the art.

When a patient catheterized with a urinary catheter requires a urine sample, the urine sample is collected in one of a number of different ways using one of a variety of different products to collect the urine sample. Typically, the urine sample is collected using a "clean" procedure, which is not aseptic and, thus, does not avoid contamination of the urine sample. It has been determined that such variation in urine sampling including where the urine sample is taken from a collection system, how the urine-sampling area is cleaned, what device is used to take the urine sample, and how the urine sample is transferred to the lab can lead to an increased risk of false-positive urine cultures. This, in turn, can lead to incorrect CAUTI diagnoses and inappropriate antibiotic treatments. As such, this variation in urine sampling is a fundamental issue presenting an ongoing challenge to those seeking to reduce CAUTI rates to avoid CMS reimbursement penalties. In addition, Centers for Disease Control ("CDC") guidelines state urine sampling from a urinary catheter should be performed using aseptic technique.

Disclosed herein are urine-sampling kits and methods thereof that address at least the foregoing. For example, the urine-sampling kits include instructions for a step-by-step approach to aseptic urine sampling from a urinary catheter using contents of the urine-sampling kits in order to limit the variation seen with current urine-sampling practice. The urine-sampling kits provide single-kit solutions for aseptic urine sampling from urinary catheters.

A urine-sampling kit can stand alone as in a stand-alone urine-sampling kit, or a urine-sampling kit can be integrated into a catheterization kit as in a combination catheterization-and-urine-sampling kit. Description for urine-sampling kits applies to both types of sampling kits; however, a separate section is included for combination catheterization-and-urine-sampling kits as such kits include additional catheterization contents and arrangements thereof.

### Urine-sampling kits

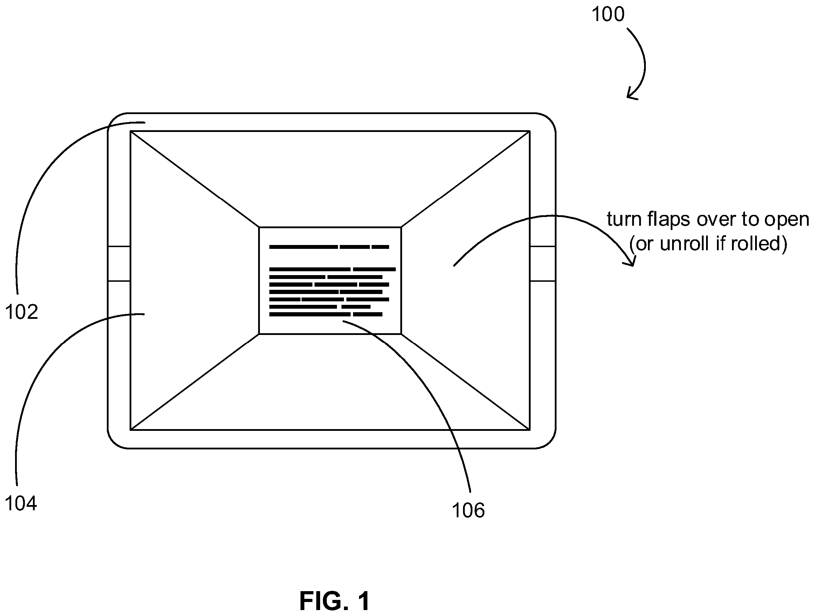

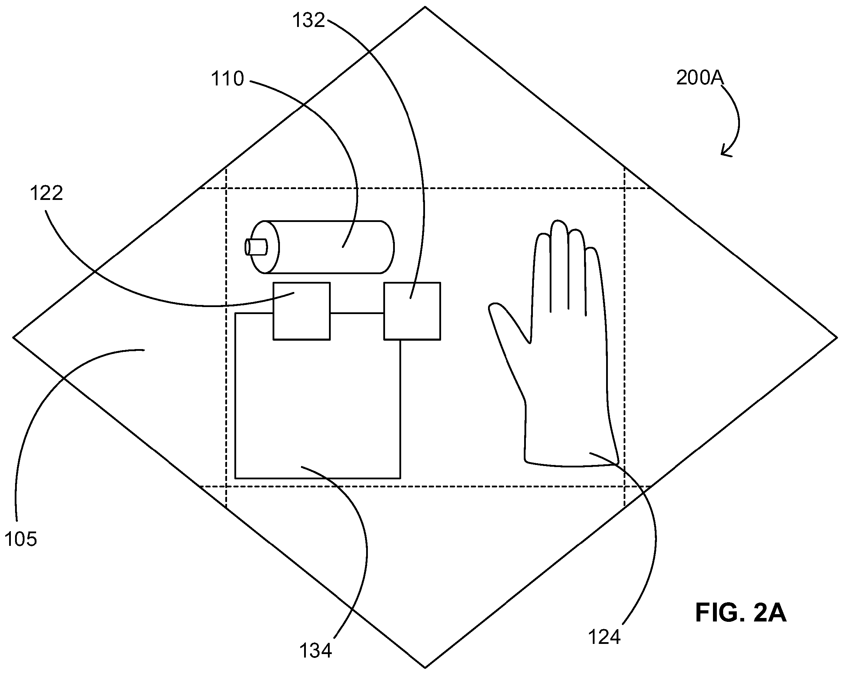

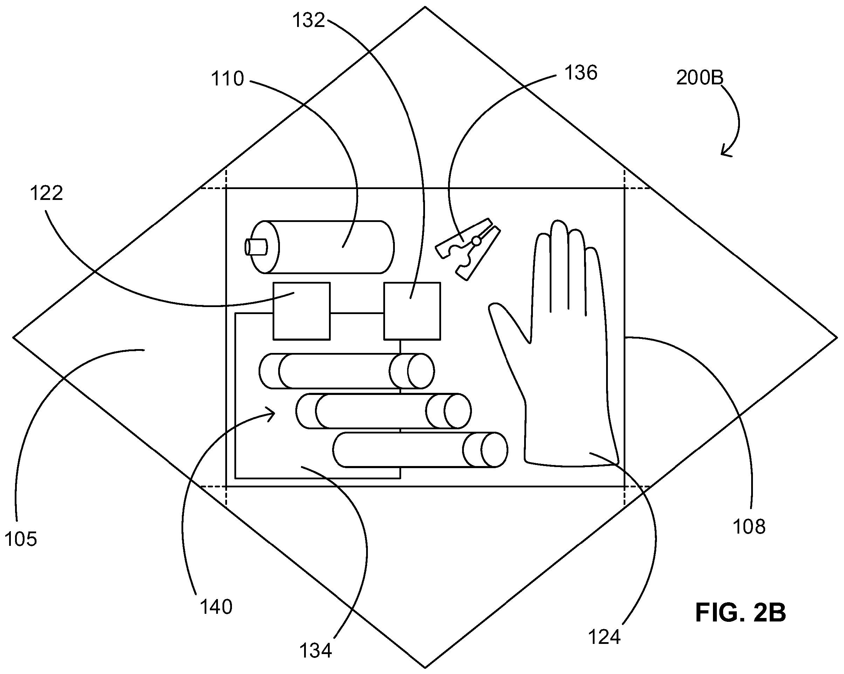

FIG. 1 provides a schematic illustrating an unopened urine-sampling kit 100 in accordance with some embodiments. In addition to the urine-sampling kit 100 representing an unopened urine-sampling kit as shown in FIG. 1, the urine-sampling kit 100 also generically refers to a urine-sampling kit disclosed herein such as either urine-sampling kit of urine-sampling kits 200A and 200B respectively of FIGS. 2A and 2B. Context determines the meaning.

As shown, the urine-sampling kit 100 can include an outer packaging 102 for the urine-sampling kit 100, a wrap or wrapping 104 about contents of the urine-sampling kit 100, and information-providing materials 106. The information-providing materials 106 can include one or more products labels, brochures, general instructions, directions for use, patient or family education cards, sticker sheets (for procedure-related labeling), or the like.

The outer packaging 102 can be configured to protect the urine-sampling kit 100 from damage and loss of the components of the urine-sampling kit 100 from a point of manufacturing the urine-sampling kit 100 to a point of using the urine-sampling kit 100. The outer packaging 102 can be a plastic such as high-density polyethylene ("HDPE"), low-density polyethylene ("LDPE"), liner low-density polyethylene ("LLDPE"), medium-density polyethylene ("MDPE"), polyethylene terephthalate ("PET"), polypropylene ("PP"), Tyvek^{®}, or the like.

The wrap or wrapping 104 about the contents of the urine-sampling kit 100 can include CSR wrap wrapped or rolled around the contents of the urine-sampling kit 100. The CSR wrap can be configured to preserve a sterile state of the contents of the urine-sampling kit 100 while the CSR wrap is wrapped around the contents of the urine-sampling kit 100.

The information-providing materials 106 can include a label, a card, or a band about the wrap or wrapping 104 including instructions for one or more steps of a set of step-by-step instructions incorporated into the urine-sampling kit 100 for preparing a patient for urine sampling and taking a urine sample. Additional steps of the step-by-step instructions are provided by way of more of the information-providing material 106 within the urine-sampling kit 100.

While not shown in FIG. 1, the urine-sampling kit 100 can include one or more hand cleansing or antisepsis products within the outer packaging 102 but on top of the wrap or wrapping 104 along with the information-providing materials 106. Such hand cleansing or antisepsis products include one or more packages of antiseptic wipes, hand sanitizer, or the like.

FIG. 2A provides a schematic illustrating a first unwrapped urine-sampling kit 200A in accordance with some embodiments. The urine-sampling kit 200A can be the urine-sampling kit 100 of FIG. 1 with the outer packaging 102 removed and flaps of the wrap or wrapping 104 about the contents of the urine-sampling kit 100 turned over as shown in FIG. 1.

As shown, the urine-sampling kit 200A can include a number of sterile contents of the urine-sampling kit 200A disposed over a sterile working surface 105, which is an inner surface of the wrap or wrapping 104. The contents can include, but are not limited to, a sampling-port access device 110, a package of hand sanitizer 122, a pair of sterile gloves 124, and a packaged antiseptic-saturated material having a cleansing form 132 that is configured for cleansing such as a packaged antiseptic wipe, towelette, or swabstick. Contents of the urine-sampling kit including the sampling-port access device 110, the package of hand sanitizer 122, the pair of sterile gloves 124, and the packaged antiseptic-saturated material having the cleansing form 132 can be packaged in accordance with step-by-step instructions for aseptic urine sampling. Some of the instructions can be exposed or unveiled as one or more pieces of the contents are removed from the urine-sampling kit 100, which can include variations in the urine sampling.

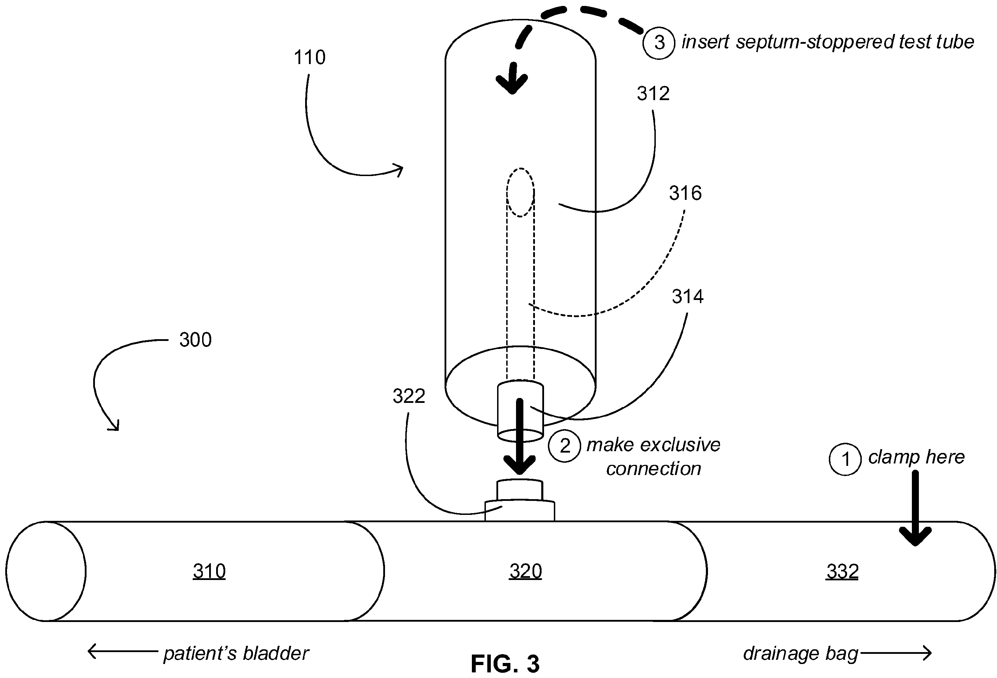

The sampling-port access device 110 can include a barrel 312, a tip 314 at an end of the barrel 312 configured to fluidly connect the sampling-port access device 110 to a urine-sampling port 322 of a catheter assembly 300, and a hollow needle 316 coaxial with the barrel 312. The needle 316 is fluidly connected to but directed away from the tip 314 of the barrel. (*See* FIG. 3 for the barrel 312, the tip 314, and the needle 316 of the sampling port access device 110 and the urine-sampling port 322 of the catheter assembly 300.)

The urine-sampling kit 200A can further include a fenestrated drape 134 for placement over a patient when preparing to take a urine sample and while taking the urine sample. A fenestration of the fenestrated drape can be shaped and sized to allow access to the urine-sampling port 322 of the catheter assembly 300 when the fenestrated drape 134 is draped over the patient. The fenestrated drape 134 is configured to provide a protective covering that prevents contamination of the urine-sampling port 322 by, for example, fecal matter or pathogens from another source, thereby reducing urine-sample contamination, which can otherwise lead to false-positive results. The fenestrated drape 134 can also lead to a decreased risk of CAUTI due to migration of microorganisms up an external surface of the urinary catheter.

FIG. 2B provides a schematic illustrating a second unwrapped urine-sampling kit 200B in accordance with some embodiments. The urine-sampling kit 200B can be the urine-sampling kit 100 of FIG. 1 with the outer packaging 102 removed and the flaps of the wrap or wrapping 104 about the contents of the urine-sampling kit 100 turned over as shown in FIG. 1.

As shown, the urine-sampling kit 200B can include the number of sterile contents of the urine-sampling kit 200A disposed over the sterile working surface 105 as well as additional contents to the urine-sampling kit 200A. The additional contents include one or more septum-stoppered test tubes 140. The additional contents can include, but are not limited to, the fenestrated drape 134, and a tubing clamp 136. In addition, the contents of any urine-sampling kit disclosed herein can be in a molded tray 108 such as that shown in FIG. 2B.

The molded tray 108 can include one or more compartments configured to hold the contents of the urine-sampling kit 100. The step-by-step instructions for aseptic urine sampling with the urine-sampling kit 100 such as in the information-providing materials 106 can be distributed among the one or more compartments. Again, some of the instructions can even be exposed or unveiled as one or more pieces of the contents are removed from the urine-sampling kit 100. In such instances, the instructions can be molded into or written onto the molded tray 108.

The molded tray 108 can be molded from, for example, high-impact polystyrene ("HIPS"). Due to the molding process, the molded tray 108 has one or more characteristics of being molded, which can include slight imperfections that do not affect the performance of the molded tray 108.

The tubing clamp 136 can be configured to clamp drainage tubing 332 between a urine-sampling port 322 and a drainage bag of a catheter assembly 300. (*See* FIG. 3 for the drainage tubing 332, the urine-sampling port 322, and the catheter assembly 300.) Clamping the drainage tubing 332 with the tubing clamp 136 allows urine to back up into the urine-sampling port 322 for urine sampling.

Each test tube of the one or more test tubes 140 is stoppered and evacuated to have an internal pressure less than atmospheric pressure. Furthermore, each test tube of the one or more test tubes can be round-bottomed or conical to accommodate different analytical instrumentation. Moreover, each test tube of the one or more test tubes can have an outer diameter commensurate with or smaller than an inner diameter of the barrel 312 of the sampling-port access device 110. Sized as such, each test tube is configured to slide into the barrel 312 sampling-port access device 110 to pierce the septum stopper of the test tube with the needle 316 of the sampling-port access device 110 for urine sampling. *(See* FIG. 3 for the barrel 312, the tip 314, and the needle 316 of the sampling port access device 110.)

Each test tube of the one or more test tubes is independently configured to include therein a formulation for urinalysis, or a formulation for microbiological analysis. Each test tube of the one or more test tubes may also include no additives or preservatives. The formulation for urinalysis includes chlorhexidine, ethylparaben, and sodium propionate to ensure sample integrity for up to at least 72 hours at room temperature while preventing overgrowth of existing microorganisms. The formulation for microbiological analysis includes boric acid, sodium formate, and sodium borate to maintain sample integrity for up to at least 48 hours at room temperature while preventing overgrowth or false positives without causing toxicity to existing microorganisms.

In view of the foregoing, the urine-sampling kit 100 can include the molded tray 108, CSR wrap, and the outer packaging 102. The molded tray 108 can include one or more compartments configured to hold the contents of the urine-sampling kit 100, which can include, but is not limited to, the sampling-port access device 110, the package of hand sanitizer 122, the pair of sterile gloves 124, the packaged antiseptic-saturated material having the cleansing form 132, the fenestrated drape 134, the tubing clamp 136, the one or more septum-stoppered test tubes 140, or a combination thereof. The information-providing materials 106 are also included in the one or more compartments and, optionally, even molded into or written onto the molded tray 108. The CSR wrap can be configured to preserve a sterile state of the contents of the urine-sampling kit 100 while the CSR wrap is wrapped around the molded tray 108 and the contents of the urine-sampling kit 100. The outer packaging 102 of the urine-sampling kit 100 can be configured to protect the urine-sampling kit 100 from damage and loss of the components of the urine-sampling kit 100 from a point of manufacturing the urine-sampling kit 100 to a point of using the urine-sampling kit 100.

### Combination catheterization-and-urine-sampling kits

As discussed above, a urine-sampling kit can stand alone or be integrated into a catheterization kit to form a combination catheterization-and-urine-sampling kit. Such a catheterization-and-urine-sampling kit can include the urine-sampling kit 100 set forth above, or a variation thereof, in combination with features of the catheterization kits disclosed in the following copending patent applications titled "Catheter Insertion Tray with Integrated Instructions:" U.S. Patent Application No. 15/029,613, filed as a U.S. national stage application from International Patent Application No. PCT/US14/60963, filed October 16, 2014, and published as US 2016-0228676, and U.S. Patent Application No. 15/487,297, filed April 13, 2017, and published as US 2017-0216558.

The catheterization-and-urine-sampling kit includes a set of catheterization components and a set of urine-sampling components. As shown in FIG. 3, the set of catheterization components include a pre-connected catheter assembly 300 including a urinary catheter 310 (e.g., a Foley catheter), drainage tubing 332, and a connector 320 fluidly connecting the urinary catheter 310 to the drainage tubing 332. The connector 320 includes a urine-sampling port 322. The set of urine-sampling components include the sampling port access device 110, the package of hand sanitizer 122, the pair of sterile gloves 124, and the packaged antiseptic-saturated material having the cleansing form 132 as shown in FIGS. 2A and 2B and described in association therewith. The set of urine-sampling components further include the one or more septum-stoppered test tubes 140 shown in FIG. 2B and described in association therewith. Contents of the urine-sampling kit 100 including the sampling-port access device 110, the package of hand sanitizer 122, the pair of sterile gloves 124, and the packaged antiseptic-saturated material having the cleansing form 132 are packaged in accordance with step-by-step instructions for aseptic urine sampling. Providing the contents of the catheterization kit together with the contents of urine-sampling kit 100 makes effective use of an exclusive connection between the sampling-port access device 110 and the urine-sampling port 322 of the catheter assembly 300 providing further assurance urine samples are collected aseptically without equipment-mismatching errors that might contaminate the urine samples.

The catheterization-and-urine-sampling kit can further include a first fenestrated drape and a second fenestrated drape such as the fenestrated drape 134. The first fenestrated drape can be provided for placement over a patient. A fenestration of the first fenestrated drape can be shaped and sized to allow access to the patient for insertion of the urinary catheter 310 when the first fenestrated drape is draped over the patient. The second fenestrated drape (e.g., the fenestrated drape 134) can also be provided for placement over the patient. A fenestration of the second fenestrated drape can be shaped and sized to allow access to the urine-sampling port 322 when the second fenestrated drape is draped over the patient. The catheterization-and-urine-sampling kit can further include a protective drape to protect the urine-sampling port 322 prior to a first urine-sampling event and between urine sampling events.

The catheterization-and-urine-sampling kit can further include a bedsheet clip for securing the drainage tubing 332 to one or more bedsheets. The bedsheet clip can also be configured to clamp the drainage tubing 332 between the urine-sampling port 322 and a drainage bag of the catheter assembly 300 obviating a need for a separate tubing clamp such as the tubing clamp 136 shown in FIG. 2B and described in association therewith. That said, the tubing clamp 136 can be provided in the catheterization-and-urine-sampling kit to allow for securing the drainage tubing 332 to one or more bedsheets with the bedsheet clip while clamping the drainage tubing 332 with the tubing clamp 136. Clamping the drainage tubing 332 with the bedsheet clip allows urine to back up into the connector 320 and the urine-sampling port 322 for urine sampling.

The catheterization-and-urine-sampling kit can further include a molded tray, a wrap or wrapping such as CSR wrap, an outer packaging, or a combination thereof like that shown in FIGS. 1, 2A, and 2B and described in association therewith. The molded tray can include one or more compartments configured to hold the contents of the catheterization-and-urine-sampling kit, which can include, but is not limited to, the set of catheterization components (e.g., the pre-connected catheter assembly 300 including the urinary catheter 310, the drainage tubing 332, and the connector 320 fluidly, the first fenestrated drape, the bedsheet clip, or a combination thereof) and the set of urine-sampling components (e.g., the sampling-port access device 110, the package of hand sanitizer 122, the pair of sterile gloves 124, the packaged antiseptic-saturated material having the cleansing form 132, the second fenestrated drape, the tubing clamp 136, the one or more septum-stoppered test tubes 140, or a combination thereof). Information-providing materials such as the information-providing materials 106 can also be included in the one or more compartments of the molded tray and, optionally, even molded into or written onto the molded tray. The CSR wrap can be configured to preserve a sterile state of the contents of the catheterization-and-urine-sampling kit while the CSR wrap is wrapped around the molded tray and the contents of the catheterization-and-urine-sampling kit. The outer packaging of the catheterization-and-urine-sampling kit can be configured to protect the catheterization-and-urine-sampling kit from damage and loss of the components of the catheterization-and-urine-sampling kit from a point of manufacturing the catheterization-and-urine-sampling kit to a point of using the catheterization-and-urine-sampling kit.

### Exclusive connection of the sampling-port access device

FIG. 3 provides a schematic illustrating an exclusive connection between the sampling-port access device 110 and the urine-sampling port 322 of the connector 320 in accordance with some embodiments.

As shown in FIG. 3, the sampling-port access device 110 includes a tip 314 at an end of a barrel 312 configured to fluidly and exclusively connect the sampling-port access device 110 to the urine-sampling port 322 of the catheter assembly 300. Each of the urine sampling port of the connector 320 and the tip 314 of the sampling-port access device 110 has a complementary thread pattern to the other for locking the sampling-port access device 110 onto the urine-sampling port 322. Embodiments of urine-sampling port devices with an exclusive connection are disclosed in U.S. Provisional Application No. 62/674,956, filed on May 22, 2018, and titled "Systems For Aseptic Urine Sampling And Methods Thereof,".

### Methods not encompassed by the scope of the claims

Contents of the urine-sampling kit 100 or the catheterization-and-urine- sampling kit are packaged in accordance with step-by-step instructions for a method of aseptic urine sampling. Such step-by-step instructions include how to aseptically prepare a patient for taking a urine sample, take the urine sample from the patient by way of from the urine-sampling port 322 of the catheter assembly 300, and finish taking the urine sample from the patient, thereby reducing healthcare-associated infections ("HAIs") such as CAUTIs. The method for aseptic urine sampling will be described in terms of using the urine-sampling kit 100 with the understanding that the method also applies to urine sampling with the catheterization-and- urine-sampling kit.

The method for aseptic urine sampling includes, but is not limited to, removing an outer packaging from a urine-sampling kit such as the outer packaging 102 from the urine sampling kit 100, aseptically unwrapping the urine-sampling kit 100, cleaning a urine-sampling port of a catheter assembly such as the urine-sampling port 322 of the catheter assembly 300, optionally, either before or after aseptically unwrapping the urine-sampling kit 100, fluidly connecting a sampling-port access device such as the sampling-port access device 110 to the urine-sampling port 322, and aspirating a urine sample from the urine-sampling port 322.

Removing the outer packaging 102 from the urine-sampling kit 100 includes exposing one or more steps of the set of step-by-step instructions incorporated into the urine-sampling kit 100 for the urine sampling. Any additional materials such as brochures or education-oriented cards of the information-providing material 106 about the contents of the urine-sampling kit 100 can be set aside while the outer packaging 102 and any additional packaging can be properly discarded. Removing the outer packaging 102 from the urine-sampling kit 100 can further include exposing the hand sanitizer 122 for hand antisepsis and the packaged antiseptic-saturated material having the cleansing form 132.

Unwrapping the urine-sampling kit 100 such as by unrolling a roll of CSR wrap around the contents of the urine-sampling kit 100 or turning corners of CSR wrap over (*see* FIG. 1) includes forming the sterile working surface 105 of an inner surface of the CSR wrap for the urine sampling. Unwrapping the urine-sampling kit 100 also includes exposing additional steps of the step-by-step instructions as well as sterile contents of the urine-sampling kit 100 disposed over the sterile working surface 105. The sterile contents of the urine-sampling kit include those packaged within the wrap or wrapping 104 such as at least the sampling-port access device 110 and the packaged antiseptic-saturated material having the cleansing form 132.

Cleaning the urine-sampling port or the catheter assembly includes cleaning the urine-sampling port with the antiseptic-saturated material having the cleansing form.

The method for aseptic urine sampling can further include obtaining the one or more septum-stoppered test tubes 140 and sliding at least one test tube of the one or more test tubes 140 into the barrel 312 of the sampling-port access device 110. The one or more septum-stoppered test tubes 140 are optionally obtained from the urine-sampling kit 100 if provided therein. If not provided in the urine-sampling kit 100, the one or more test tubes 140 can be obtained from, for example, a central supply department. Sliding the at least one test tube of the one or more test tubes 140 into the barrel 312 of the sampling-port access device 110 pierces the septum stopper of the test tube with the hollow needle 316 disposed in the barrel 312 of the sampling-port access device 110. Once the needle 316 pierces the septum stopper of the test tube, urine is automatically aspirated into the test tube from the urine-sampling port 322 due to the less-than-atmospheric internal pressure of the test tube to provide a urine sample.

The method for aseptic urine sampling can further include clamping the drainage tubing 332 of the catheter assembly 300 with the tubing clamp 136 (or the bedsheet clip of the catheterization-and-urine-sampling kit) between the urine-sampling port 322 and the drainage bag of the catheter assembly 300. Clamping the drainage tubing 332 with the tubing clamp 136 (or the bedsheet clip) allows urine to back up into the urine-sampling port 322 for urine sampling.

The method for aseptic urine sampling can further include aseptically removing the package of hand sanitizer 122 provided in the urine-sampling kit 100 from the sterile working surface 105, if provided as such, and subsequently using the hand sanitizer 122 for hand antisepsis. In addition, the method for aseptic urine sampling can further include removing the pair of sterile gloves 124 provided in the urine-sampling kit 100 from the sterile working surface 105 and subsequently donning the pair of sterile gloves 124.

The method for aseptic urine sampling can further include handwashing or hand antisepsis and subsequently donning a pair of exam gloves before removing the outer packaging 102 from the urine-sampling kit 100. Because the pair of exam gloves has a potential to get contaminated by the outer packaging 102 of the urine-sampling kit 100, the method for aseptic urine sampling can further include removing the pair of exam gloves after removing the outer packaging 102 from the urine-sampling kit 100. Optionally following additional hand washing or hand antisepsis, the method for aseptic urine sampling can further include aseptically unwrapping the urine-sampling kit 100 to expose the sterile contents of the urine-sampling kit.

Following the method for aseptic urine sampling, the urine sample or samples in the one or more test tubes can be labeled, optionally refrigerated, and subsequently put in a transport pouch for transport to a lab for urinalysis or microbiological analysis.

The urine-sampling kit 100 and the catheterization-and-urine-sampling kit can be manufactured by first manufacturing the contents of the urine-sampling kit 100 and the catheterization-and-urine-sampling kit. A molded tray such as the molded tray 108 can be molded in accordance with the urine-sampling kit 100 or the catheterization-and-urine-sampling kit and filled with the contents thereof. The molded tray can be wrapped in a wrap or wrapping 104 such as CSR wrap and placed in a bag of the outer packaging 102. Once in the bag of the outer packaging 102, the entire urine-sampling kit 100 or catheterization-and-urine-sampling kit can be sterilized by way of gas sterilization and sealed in the bag.

An advantage of the urine-sampling kit 100 or the catheterization-and-urine-sampling kit and the aseptic urine-sampling method includes a standardization of clinical practice for taking urine samples from catheterized patients, thereby eliminating the risk of patient infection, contaminated samples (by sample-port contamination), and false-positive urine cultures. Furthermore, the method of aseptic urine sampling ensures compliance with CDC guidelines related thereto.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein provided that they fall under scope of the appended claims.

## Claims

1. A catheterization-and-urine-sampling kit, comprising:
a set of catheterization components, including a pre-connected catheter assembly (300) including a urinary catheter (310), drainage tubing (332), and a connector (320) fluidly connecting the urinary catheter to the drainage tubing, the connector including a urine-sampling port (322);
a sampling-port access device (110), including:
a barrel (312);
a tip (314) at an end of the barrel configured to fluidly connect the sampling-port access device (110) to the urine-sampling port (332) of the catheter assembly (300); and
a hollow needle (316) coaxial with the barrel, the needle fluidly connected to but directed away from the tip of the barrel;
a package of hand sanitizer (122);
a pair of sterile gloves (124); and
a packaged anti septic-saturated material having a cleansing form (132) configured for cleansing,
wherein contents of the urine-sampling kit including the sampling-port access device, the package of hand sanitizer, the pair of sterile gloves, and the packaged antiseptic-saturated material having the cleansing form are packaged in accordance with step-by-step instructions for aseptic urine sampling;
the catheterization-and-urine sampling kit further comprising one or more septum-stoppered test tubes (140), each test tube of the one or more test tubes having an internal pressure less than atmospheric pressure, wherein each test tube of the one or more test tubes independently includes therein a formulation for urinalysis or a formulation for microbiological analysis, wherein the formulation for urinalysis includes chlorhexidine, ethylparaben, or sodium propionate and wherein the formulation for microbiological analysis includes boric acid, sodium formate, or sodium borate.

2. The catheterization-and-urine-sampling kit of claim 1, further comprising a fenestrated drape (134) for placement over a patient, a fenestration of the fenestrated drape shaped and sized to allow access to the urine-sampling port (322) of the catheter assembly when the fenestrated drape is draped over the patient.

3. The catheterization-and-urine-sampling kit of either claim 1 or claim 2, further comprising a tubing clamp (136) configured to clamp the drainage tubing between the urine-sampling port (322) and a drainage bag of a catheter assembly, thereby allowing urine to back up into the urine-sampling port (322) for urine sampling.

4. The catheterization-and-urine-sampling kit of any claim of claims 1-3,
further comprising central supply room ("CSR") wrap, the CSR wrap configured to preserve a sterile state of the contents of the catheterization-and-urine-sampling kit while the CSR wrap is wrapped around the contents of the catheterization-and-urine-sampling kit.

5. The catheterization-and-urine-sampling kit of any claim of claims 1-4, further comprising a molded tray (108) including one or more compartments configured to hold the contents of the catheterization-and-urine-sampling
kit.

6. The catheterization-and-urine sampling kit of claims 1 to 5, further comprising an outer packaging (102) of the catheterization-and-urine-sampling kit, the outer packaging configured to protect the catheterization-and-urine-sampling kit from damage and loss of the components of the catheterization-and-urine-sampling kit from a point of manufacturing the catheterization-and-urine-sampling kit to a point of using the catheterization-and-urine-sampling kit.

7. The catheterization-and-urine-sampling kit of claims 1-5, wherein each of the urine-sampling port (322) of the connector (320) and the tip (314) of the sampling-port access device (110) has a complementary thread pattern to the other for locking the sampling-port access device onto the urine-sampling port.

8. The catheterization-and-urine-sampling kit of claim 2, further comprising:
a second fenestrated drape (134) for placement over a patient, a fenestration of the second fenestrated drape shaped and sized to allow access to the patient for insertion of the urinary catheter (310) when the second fenestrated drape is draped over the patient.

9. The catheterization-and-urine-sampling kit of any claim of claims 1-8, further comprising a bedsheet clip for securing the drainage tubing to one or more bedsheets, the bedsheet clip also configured to clamp the drainage tubing (332) between the urine-sampling port (322) and a drainage bag of the catheter assembly, thereby allowing urine to back up into the urine-sampling port (322) for urine sampling.

10. The catheterization-and-urine-sampling kit of any claim of claims 1 -9, wherein each test tube (140) of the one or more test tubes has an outer diameter commensurate with or smaller than an inner diameter of the barrel (312) of the sampling-port access device (110), thereby allowing each test tube to slide into the barrel to pierce the septum stopper of the test tube with the needle (316) of the sampling-port access device for urine sampling.

## Patentansprüche

1. Katheterisierungs- und Urinprobenentnahme-Kit, umfassend:
einen Satz von Katheterisierungskomponenten, einschließlich einer vorverbundenen Katheteranordnung (300) einschließlich eines Harnkatheters (310), eines Drainageschlauchs (332) und eines Verbinders (320), der den Harnkatheter fluidisch mit dem Drainageschlauch verbindet, wobei der Verbinder einen Urinprobenentnahmeanschluss (322) einschließt;
eine Probenentnahmeanschlusszugangsvorrichtung (110), einschließend:
einen Zylinder (312);
eine Spitze (314) an einem Ende des Zylinders, die konfiguriert ist, um die Probenentnahmeanschlusszugangsvorrichtung (110) fluidisch mit dem Urinprobenentnahmeanschluss (332) der Katheteranordnung (300) zu verbinden; und
eine Hohlnadel (316), die koaxial zum Zylinder ist, wobei die Nadel fluidisch
mit der Spitze des Zylinders verbunden ist, aber von dieser weggerichtet ist;
eine Verpackung eines Handdesinfektionsmittels (122);
ein Paar steriler Handschuhe (124); und
ein verpacktes, antiseptisch gesättigtes Material mit einer Reinigungsform (132), die für die Reinigung konfiguriert ist,
wobei der Inhalt des Urinprobenentnahme-Kits einschließlich der Probenentnahmeanschlusszugangsvorrichtung, der Verpackung des Handdesinfektionsmittels, des Paares steriler Handschuhe und des verpackten antiseptisch gesättigten Materials mit der Reinigungsform nach einer schrittweisen Anleitung für die Urinprobenentnahme verpackt werden;
wobei das Katheterisierungs- und Urinprobenentnahme-Kit weiter ein oder mehrere mit Septumstopfen versehene Reagenzgläser (140) umfasst, wobei jedes Reagenzglas des einen oder der mehreren Reagenzgläser einen Innendruck aufweist, der geringer als Atmosphärendruck ist, wobei jedes Reagenzglas des einen oder der mehreren Reagenzgläser unabhängig darin eine Formulierung für die Urinanalyse oder eine Formulierung für die mikrobiologische Analyse einschließt, wobei die Formulierung für die Urinanalyse Chlorhexidin, Ethylparaben oder Natriumpropionat einschließt und wobei die Formulierung für die mikrobiologische Analyse Borsäure, Natriumformiat oder Natriumborat einschließt.

2. Katheterisierungs- und Urinprobenentnahme-Kit nach Anspruch 1, weiter umfassend ein gefenstertes Abdecktuch (134) zur Platzierung über einem Patienten, wobei eine Fenestration des gefensterten Abdecktuchs so geformt und bemessen ist, dass es Zugang zu dem Urinprobenentnahmeanschluss (322) der Katheteranordnung ermöglicht, wenn das gefensterte Abdecktuch über dem Patienten drapiert ist.

3. Katheterisierungs- und Urinprobenentnahme-Kit nach Anspruch 1 oder Anspruch 2, weiter umfassend eine Schlauchklemme (136), die konfiguriert ist, um den Drainageschlauch zwischen dem Urinprobenentnahmeanschluss (322) und einem Drainagebeutel einer Katheteranordnung zu klemmen, wodurch es ermöglicht wird, dass Urin in den Urinprobenentnahmeanschluss (322) zur Urinprobenentnahme zurückkehrt.

4. Katheterisierungs- und Urinprobenentnahme-Kit nach einem der Ansprüche 1 - 3, weiter umfassend eine Umhüllung des zentralen Versorgungsraums ("CSR"), wobei die CSR-Umhüllung konfiguriert ist, einen sterilen Zustand des Inhalts des Katheterisierungs- und Urinprobenentnahme-Kits zu erhalten, während die CSR-Umhüllung um den Inhalt des Katheterisierungs- und Urinprobenentnahme-Kits gewickelt ist.

5. Katheterisierungs- und Urinprobenentnahme-Kit nach einem der Ansprüche 1 - 4, weiter umfassend eine geformte Schale (108), die ein oder mehrere Kompartimente einschließt, die konfiguriert sind, um den Inhalt des Katheterisierungs- und Urinprobenentnahme-Kits aufzunehmen.

6. Katheterisierungs- und Urinprobenentnahme-Kit nach den Ansprüchen 1 bis 5, weiter umfassend eine Außenverpackung (102) des Katheterisierungs- und Urinprobenentnahme-Kits, wobei die Außenverpackung konfiguriert ist, um das Katheterisierungs- und Urinprobenentnahmekit vor Beschädigung und Verlust der Komponenten des Katheterisierungs- und Urinprobenentnahme-Kits von einem Punkt der Herstellung des Katheterisierungs- und Urinprobenentnahme-Kits bis zu einem Punkt der Verwendung des Katheterisierungs- und Urinprobenentnahme-Kits zu schützen.

7. Katheterisierungs- und Urinprobenentnahme-Kit nach den Ansprüchen 1 - 5, wobei sowohl der Urinprobenentnahmeanschluss (322) des Verbinders (320) als auch die Spitze (314) der Probenentnahmeanschlusszugangsvorrichtung (110) ein komplementäres Gewindemuster zu dem anderen aufweist, um die Probenentnahmeanschlusszugangsvorrichtung an dem Urinprobenentnahmeanschluss zu verriegeln.

8. Katheterisierungs- und Urinprobenentnahme-Kit nach Anspruch 2, weiter umfassend:
ein zweites gefenstertes Abdecktuch (134) zum Platzieren über einem Patienten, wobei eine Fenestration des zweiten gefensterten Abdecktuchs so geformt und bemessen ist, dass es Zugang zum Patienten zum Einführen des Harnkatheters (310) ermöglicht, wenn das zweite gefensterte Abdecktuch über dem Patienten drapiert ist.

9. Katheterisierungs- und Urinprobenentnahme-Kit nach einem der Ansprüche 1 - 8, weiter umfassend einen Bettlakenclip zum Befestigen des Drainageschlauchs an einem oder mehreren Bettlaken, wobei der Bettlakenclip auch konfiguriert ist, um den Drainageschlauch (332) zwischen dem Urinprobenentnahmeanschluss (322) und einem Drainagebeutel der Katheteranordnung zu klemmen, wodurch Urin in den Urinprobenentnahmeanschluss (322) zur Urinprobenahme gelangen kann.

10. Katheterisierungs- und Urinprobenentnahme-Kit nach einem der Ansprüche 1 - 9, wobei jedes Reagenzglas (140) des einen oder der mehreren Reagenzgläser einen Außendurchmesser aufweist, der einem Innendurchmesser des Zylinders (312) der Probenentnahmeanschlusszugangsvorrichtung (110) entspricht oder kleiner als dieser ist, wodurch es jedem Reagenzglas ermöglicht wird, in den Zylinder zu gleiten, um den Septumstopfen des Reagenzglases mit der Nadel (316) der Probenentnahmeanschlusszugangsvorrichtung zur Urinentnahme zu durchstechen.

## Revendications

1. Kit de cathétérisme et de prélèvement d'échantillons d'urine, comprenant :
un ensemble de composants de cathétérisme, incluant un ensemble (300) sonde pré-raccordé incluant une sonde urinaire (310), une tubulure de drainage (332), et un raccord (320) reliant de manière fluidique la sonde urinaire à la tubulure de drainage, le raccord incluant un point de prélèvement d'urine (322) ;
un dispositif d'accès au point de prélèvement (110), incluant :
un cylindre (312) ;
un embout (314) à une extrémité du cylindre, conçu pour relier fluidiquement le dispositif d'accès au point de prélèvement (110) au point de prélèvement d'urine (332) de l'ensemble (300) sonde ; et
une aiguille (316) creuse coaxiale avec le cylindre, l'aiguille étant reliée fluidiquement à l'embout du cylindre, mais dirigée à l'opposé de celui-ci ;
un paquet de désinfectant pour les mains (122) ;
une paire de gants stériles (124) ; et
un matériau imprégné d'antiseptique emballé présentant une forme de nettoyage (132) conçue pour le nettoyage,
dans lequel le contenu du kit de prélèvement d'urine, incluant le dispositif d'accès au point de prélèvement, l'emballage de désinfectant pour les mains, la paire de gants stériles, et le matériau imprégné d'antiseptique emballé présentant la forme de nettoyage, est emballé conformément à des instructions étape par étape pour le prélèvement d'urine aseptique ;
le kit de cathétérisme et de prélèvement d'urine comprenant en outre un ou plusieurs tubes à essai (140) bouchés par un septum, chaque tube à essai du ou des tubes à essai présentant une pression interne inférieure à la pression atmosphérique, dans lequel chaque tube à essai du ou des tubes à essai inclut indépendamment une formulation pour l'analyse d'urine ou une formulation pour l'analyse microbiologique, dans lequel la formulation pour l'analyse d'urine inclut de la chlorhexidine, de l'éthylparabène, ou du propionate de sodium, et dans lequel la formulation pour l'analyse microbiologique inclut de l'acide borique, du formiate de sodium, ou du borate de sodium

2. Kit de cathétérisme et de prélèvement d'urine selon la revendication 1, comprenant en outre un champ fenêtré (134) à placer sur un patient, une fenestration du champ fenêtré étant formée et dimensionnée pour permettre l'accès au point de prélèvement d'urine (322) de l'ensemble sonde lorsque le champ fenêtré est positionné sur le patient.

3. Kit de cathétérisme et de prélèvement d'urine selon la revendication 1 ou la revendication 2, comprenant en outre une pince pour tube (136) conçue pour serrer la tubulure de drainage entre le point de prélèvement d'urine (322) et une poche de drainage d'un ensemble sonde, permettant ainsi à l'urine de remonter dans le point de prélèvement d'urine (322) pour le prélèvement d'urine.

4. Kit de cathétérisme et de prélèvement d'urine selon l'une quelconque des revendications 1 à 3, comprenant en outre une enveloppe de service central d'approvisionnement ("CSR"), l'enveloppe CSR étant conçue pour préserver un état stérile du contenu du kit de cathétérisme et de prélèvement d'urine pendant que l'enveloppe CSR est enroulée autour du contenu du kit de cathétérisme et de prélèvement d'urine.

5. Kit de cathétérisme et de prélèvement d'urine selon l'une quelconque des revendications 1 à 4, comprenant en outre un plateau moulé (108) incluant un ou plusieurs compartiments conçus pour contenir le contenu du kit de cathétérisme et de prélèvement d'échantillons d'urine.

6. Kit de cathétérisme et de prélèvement urinaire selon les revendications 1 à 5, comprenant en outre un emballage extérieur (102) du kit de cathétérisme et de prélèvement d'urine, l'emballage extérieur étant conçu pour protéger le kit de cathétérisme et de prélèvement d'urine des dommages et de la perte des composants du kit de cathétérisme et de prélèvement d'urine entre un moment de fabrication du kit de cathétérisme et de prélèvement d'urine et un moment d'utilisation du kit de cathétérisme et de prélèvement d'urine.

7. Kit de cathétérisme et de prélèvement d'urine selon les revendications 1 à 5, dans lequel le point de prélèvement d'urine (322) du raccord (320) et l'embout (314) du dispositif d'accès au point de prélèvement (110) possèdent chacun un motif de filetage complémentaire pour verrouiller le dispositif d'accès au point de prélèvement sur le point de prélèvement d'urine.

8. Kit de cathétérisme et de prélèvement d'urine selon la revendication 2, comprenant en outre :
un second champ fenêtré (134) destiné à être placé sur un patient, une fenestration du second champ fenêtré étant formée et dimensionnée pour permettre l'accès au patient pour l'insertion de la sonde urinaire (310) lorsque le second champ fenêtré est positionné sur le patient.

9. Kit de cathétérisme et de prélèvement d'urine selon l'une quelconque des revendications 1 à 8, comprenant en outre un clip de drap de lit pour fixer la tubulure de drainage à un ou plusieurs draps de lit, le clip de drap de lit étant également conçu pour serrer la tubulure de drainage (332) entre le point de prélèvement d'urine (322) et une poche de drainage de l'ensemble sonde, permettant ainsi à l'urine de remonter dans le point de prélèvement d'urine (322) pour le prélèvement d'urine.

10. Kit de cathétérisme et de prélèvement d'urine selon l'une quelconque des revendications 1 à 9, dans lequel chaque tube à essai (140) du ou des tubes à essai a un diamètre extérieur correspondant ou inférieur au diamètre intérieur du cylindre (312) du dispositif d'accès au point de prélèvement (110), permettant ainsi à chaque tube à essai de glisser dans le cylindre pour percer le bouchon de septum du tube à essai avec l'aiguille (316) du dispositif d'accès au point de prélèvement pour le prélèvement d'urine.
